(19) **Europäisches Patentamt**
**European Patent Office**
**Office européen des brevets**

(11) **EP 3 370 612 B1**

(12) **EUROPEAN PATENT SPECIFICATION**

(45) Date of publication and mention
of the grant of the patent:
**22.05.2019 Bulletin 2019/21**

(51) Int Cl.:
*A61B 5/05* (2006.01)      *G01R 33/12* (2006.01)
*G01N 27/74* (2006.01)

(21) Application number: **16784844.9**

(22) Date of filing: **17.10.2016**

(86) International application number:
**PCT/EP2016/074912**

(87) International publication number:
**WO 2017/076627 (11.05.2017 Gazette 2017/19)**

(54) **EXAMINATION APPARATUS FOR TRACKING PERMANENTLY MAGNETIC BEADS**

UNTERSUCHUNGSVORRICHTUNG ZUR VERFOLGUNG VON PERMANENT MAGNETISCHEN KÜGELCHEN

APPAREIL D'EXAMEN POUR LE SUIVI DE BILLES MAGNÉTIQUES EN PERMANENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2015 EP 15192698**

(43) Date of publication of application:
**12.09.2018 Bulletin 2018/37**

(73) Proprietor: **Koninklijke Philips N.V.**
**5656 AE Eindhoven (NL)**

(72) Inventor: **GLEICH, Bernhard**
**5656 AE Eindhoven (NL)**

(74) Representative: **van Wermeskerken, Stephanie Christine**
**Philips Intellectual Property & Standards**
**High Tech Campus 5**
**5656 AE Eindhoven (NL)**

(56) References cited:
**WO-A1-2016/164247      US-A1- 2007 172 890**
**US-A1- 2013 157 256**

• **KNOPP T ET AL: "Model-Based Reconstruction for Magnetic Particle Imaging", IEEE TRANSACTIONS ON MEDICAL IMAGING, IEEE SERVICE CENTER, PISCATAWAY, NJ, US, vol. 29, no. 1, 1 January 2010 (2010-01-01), pages 12-18, XP011293583, ISSN: 0278-0062**

## Description

FIELD OF THE INVENTION

The invention relates to tracking permanently magnetic beads which are flowing in a channel of an object. In particular, the invention relates to an imaging apparatus, a method for tracking a plurality of permanently magnetic beads, and a computer program.

BACKGROUND OF THE INVENTION

For tomographic imaging, magnetic resonance imaging (MRI) may offer the opportunity of acquiring images without X-rays. However, the image speed of MRIs may generally be considered to be too low for the imaging of vessels, in particular for vascular applications in the heart.

One solution for imaging vessels may be via magnetic particle imaging (MPI). In magnetic particle imaging, magnetizable particles are magnetized using a drive field. By varying the drive field in time, the magnetization of the particles is changed, which causes an oscillating magnetic field that can be measured and evaluated to extract information concerning the spatial distribution of the magnetizable particles.

However, the hardware needed for MPIs may be as large and heavy as for MRIs. Moreover, there may be patients who cannot be subjected to magnetic fields used in MPIs and MRIs.

Document US 2008/0204009 A1 relates to a method of determining a spatial distribution of magnetizable particles in an examination zone. In the method, a first magnetic field is generated, which forms in the examination zone a first subzone having a relatively low magnetic field strength and a second subzone having a relatively high magnetic field strength. The position in space of the two subzones is then changed by a second magnetic field, whereby the magnetization in the examination zone is changed. Signals dependent on the change in magnetization are acquired and evaluated to extract information concerning the spatial distribution of the magnetizable particles.

US 2007/0172890 A1, US 2013/0157256 A1, and Knopp T. et al.: "Model-Based Reconstruction for Magnetic Particle Imaging", IEEE Transaction On Medical Imaging, IEEE Service Center, Piscataway, NJ, US, 29(1), 1 January 2010, pp. 12-18 disclose all magnetic particle imaging.

SUMMARY OF THE INVENTION

There may be a need to provide an apparatus and a method for improved examination of channels in an object and for and for imaging the channel. The subject-matter of the application is set out in the independent claims. Further embodiments and examples are presented in the dependent claims, the description, and the figures.

An aspect of the invention relates to an imaging apparatus (also called examination apparatus herein) for tracking a plurality of permanently magnetic beads included in a fluid flowing in a channel of an object and for imaging the channel. The examination apparatus comprises an examination zone that is configured to receive the object. In this way, the object may be located in the examination zone. The examination apparatus further comprises at least three magnetic field sensors configured for detecting a magnetic field of the permanently magnetic beads in the examination zone and configured for creating a corresponding signal. The examination apparatus further comprises an evaluation unit configured for determining temporal magnetic field variations by processing the signal created by the at least three magnetic field sensors, wherein the temporal magnetic field variations are caused by rotations of the individual beads. The evaluation unit is configured for discriminating sub-signals caused by the individual beads in the processed signal using the determined temporal magnetic field variations. Furthermore, the evaluation unit is configured for determining position information related to the individual beads using magnitudes of the sub-signals caused by the individual beads. Finally, the evaluation unit is configured for reconstructing an image of the channel based on the determined position information of the individual beads.

In other words, the imaging apparatus may be configured for tracking individual particles or beads in an object, wherein the beads are located in the object in such a way that they are rotating. The rotation of the beads may be caused by the shear forces in the fluid flowing through the channel. The tracking of the beads may be achieved by using the temporal magnetic field variations caused by their rotations which are due to the natural shear forces of the fluid in the channel, into which the beads are inserted. Thus, the evaluation unit of the imaging apparatus may be configured for separating sub-signals caused by individual rotating permanently magnetic beads from a signal generated by the magnetic field sensors. Since the temporal magnetic field variations are caused by the rotating permanently magnetic beads, there is no need for a magnetic system for generating a drive field as in MPIs. The separation/discrimination of the sub-signals from the signal of the magnetic field sensors may be carried out using the fact that each rotating permanently magnetic bead causes its own magnetic field variation. After the sub-signals are separated or discriminated, the position of the individual permanently magnetic beads may be determined by analyzing the magnitudes or strength of the sub-signals caused by the individual beads. Furthermore, the imaging apparatus is configured for imaging the channel of the object.

Thus, the imaging apparatus, the method, and the computer program described herein may be used for any imaging applications where beads may be inserted in an object such that the beads are rotating and are causing a temporally varying magnetic field. In particular, the imaging apparatus, the method, and the computer program can be used for examination applications where beads may be inserted in a flowing fluid, e.g. into blood vessels.

In this way, tomographic images of the channel of the object may be created with a high resolution, a high tissue contrast. Furthermore, the imaging apparatus may gain information regarding the fluid flow in the channel, for example shear rates and/or shear forces. There may be no need for ionizing radiation and iodine based contrast material. Furthermore, there may be no need for providing magnetic devices for creating a drive field for magnetizing the beads because the imaging apparatus is configured for detecting and discriminating permanently magnetic beads that cause magnetic field variations due to their rotations. In this way, the imaging apparatus may be much lighter than MRIs or MPIs. Moreover, the imaging apparatus may also be usable for patients who are incompatible with the fields used in MPIs and MRIs.

It shall be noted that the imaging apparatus described in the present application can be used for imaging channels of any object, wherein a fluid is flowing through a channel of the object. For example, the imaging apparatus may be used for medical or diagnostic tomographic imaging, for example for imaging human or animal bodies. However, the imaging apparatus is not limited to medical or diagnostic applications. The same also accounts to the method and the computer program described below.

According to an exemplary embodiment of the invention, the imaging apparatus further comprises a magnetic device configured for creating a magnetic gradient field in the examination zone.

The magnetic gradient field may vary in time. The magnetic gradient field may define a field of view such that only beads located within the field of view are able to rotate. In other words, the magnetic field outside the field of view may be too strong such that the beads cannot rotate outside the field of view. Furthermore, the magnetic gradient field may comprise a field free point (FFP) or a field free line (FFL). This FFP or FFL may be displaced, e.g. along a Lissajous trajectory or a Cartesian (i.e. line by line) trajectory. In this way, a bigger volume of the object may be scanned. The magnetic gradient field may be configured to create a rotation asymmetry of the beads. The rotation asymmetry may depend on the distance and the angle from the field free point or line of magnetic gradient field. The evaluation unit may be configured to use the rotation asymmetry of the beads to determine their positions. Furthermore, the magnetic gradient field may be configured to drag the beads to the wall of the channel where the rotation of the beads may be maximized.

According to a further exemplary embodiment of the invention, the magnetic device is configured for creating the magnetic gradient field in the examination zone such that the magnetic gradient field is varying in time.

According to a further exemplary embodiment of the invention, the magnetic gradient field created by the magnetic device defines a field of view within the examination zone such that rotation of beads inside the field of view is possible and is inhibited outside the field of view.

According to a further exemplary embodiment of the invention, the magnetic gradient field comprises a field free point or a field free line.

According to a further exemplary embodiment of the invention, the evaluation unit is configured to determine information regarding shear forces acting in the fluid flowing through the channel using information about the temporal magnetic field variations; and/or the evaluation unit is configured to determine positions of the permanently-magnetic beads with respect to a wall of the object using information about the temporal magnetic field variations.

A further aspect of the invention relates to an imaging apparatus described in the application comprising a plurality of permanently magnetic beads.

The plurality of permanently magnetic beads may all be of the same size and the same shape. Alternatively, the plurality of permanently magnetic beads may comprise beads of different sizes and/or shapes. In this case, the evaluation unit may be configured for discriminating temporal magnetic field variations of the individual beads using the fact that the individual beads with different size and/or shape rotate differently due to their different sizes and/or shapes. Moreover, the plurality of permanently magnetic beads may comprise flexible chains, wherein the flexible chains comprise a plurality of successively aligned permanently magnetic beads.

A further aspect of the invention relates to a method for examination a plurality of permanently magnetic beads included in fluid flowing through a channel of an object and for imaging the channel. Furthermore, the method comprises the step of placing the object in an examination zone. Subsequently, the magnetic field of the permanently magnetic beads is detected in the examination zone and a corresponding signal is created. A further step is the determination of temporal magnetic field variations of individual beads by processing the signal, which temporal magnetic field variations are caused by the individual rotating magnetic beads. Further, sub-signals caused by the individual beads in the processed signal are discriminated using the determined temporal magnetic field variations of the individual beads. Finally, position information related to the individual beads is determined by using magnitudes of the sub-signals caused by the individual beads and an image of the channel is reconstructed based on the determined position information of the individual beads.

According to an exemplary embodiment of the invention, the method may comprise the step of inserting the plurality of permanently magnetic beads in a fluid flowing in the channel of the object to be examined, wherein the magnetic beads rotate when the fluid is flowing through the channel.

As described before, the method may be for tracking a plurality of permanently magnetic beads in a fluid flowing in a channel of any object. According to an exemplary embodiment, the object may be no human or animal body. Thus, the method may not be for treatment of the human or animal body by surgery or therapy and the method may be no diagnostic method practiced on the human or animal body. According to a further exemplary embodiment, the method may not comprise the step of inserting the permanently magnetic beads into the channel of the object.

Another aspect of the invention relates to a computer program or a computer program element, which, when it is executed by a processor of an imaging apparatus, instructs the processor to carry out a method described within this application.

A further aspect of the invention relates to a computer-readable medium such as a CD-ROM on which a computer program is stored, which, when it is executed by a processor of an imaging apparatus, instructs the processor to carry out a method described within this application.

The computer program element might therefore be stored on a computer-readable medium or a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

Rephrased differently the invention may relate to an imaging apparatus, a method and a computer program for tracking permanently magnetic beads that are transported by a fluid flowing through a channel of an object. The imaging apparatus may comprise at least three magnetic field sensors and an evaluation unit. The magnetic field sensors may be configured to detect the magnetic field caused by the permanently magnetic beads. Due to shear forces acting in the fluid, the permanently magnetic beads may be rotating and the magnetic field caused by the beads may temporally vary. This temporal variation of the magnetic field may be used by the evaluation unit for discriminating sub-signals related to single beads from the overall signal generated by the magnetic field sensors. Furthermore, the evaluation unit may determine positioning information of individual beads on the basis of the discriminated sub-signals.

These and other aspects of the invention will be apparent from and elucidated with reference to the embodiments described hereinafter.

BRIEF DESCRIPTION OF THE DRAWINGS

Fig. 1 shows an examination apparatus according to an exemplary embodiment of the invention
Fig. 2 shows an examination apparatus according to a further exemplary embodiment of the invention.
Fig. 3 shows an examination apparatus according to a further exemplary embodiment of the invention.
Figs. 4A to 4J show a permanently magnetic bead rotating in a channel and the corresponding magnetic field variations.
Figs. 5A and 5B show a signal and a sub-signal separated from said signal according to exemplary embodiments of the invention.
Fig. 6 shows a magnetic field sensor and a magnetic device according to an exemplary embodiment of the invention.
Fig. 7A shows an examination apparatus comprising a measurement device for measuring a magnetic activity of an object according to an exemplary embodiment of the invention.
Fig. 7B shows an electric potential of an object and Fig. 7C shows a magnetic field caused by the currents in the object according to exemplary embodiments of the invention.
Fig. 8 shows an examination apparatus according to an exemplary embodiment of the invention.
Figs. 9A to 9D each show a plurality of permanently magnetic beads according to exemplary embodiments of the invention.

Figs. 10 to 12 show flow-charts of methods according to exemplary embodiments of the invention.

The figures are schematic and not true to scale.

DETAILED DESCRIPTION OF EMBODIMENTS

Before turning to the detailed description of exemplary embodiments, some general aspects of the examination apparatus, the method and the computer program of the present application are described.

The examination apparatus may relate to an examination apparatus configured for tomographic imaging of an object. For example, the examination apparatus may be a medical examination apparatus. The examination apparatus may be particularly configured for examination channels of an object, in which channels a fluid is flowing, e.g. vessels of a human or animal body. However, the examination apparatus may also be used for other examination applications where beads may be inserted in an object such that they are rotating.

The permanently magnetic beads may relate to small sized particles which comprise a material that is permanently magnetic. The size of the permanently magnetic beads may be between 1 μm and 20 μm. This size of beads may e.g. be appropriate for medical uses. The feature that the beads are included in the fluid may be understood in such a way that the beads are swimming in the fluid. Furthermore, the beads may be inserted in the fluid.

Tracking the permanently magnetic beads may comprise determining a position information of the permanently magnetic beads. Furthermore, tracking may comprise determining a velocity information of the permanently magnetic beads. Moreover, an image of the channel may be reconstructed on the basis of the tracking of the permanently magnetic beads.

For example, the permanently magnetic beads may have a size that is greater than 1 μm. In this way, it may be ensured that the magnetic fields of the permanently magnetic fields may be detected. However, for examining microscopic objects (e.g. objects that are much smaller than 1 cm), the beads may be smaller than 1 μm. The size of the bead may refer to an average diameter of the bead. Moreover, if the bead has the shape of an ellipsoid, the diameter of the ellipsoid along two axis of the ellipsoid may be below 5 μm and the diameter of the ellipsoid along the third axis of the ellipsoid may be smaller or greater than 5 μm. For example, the diameter of the ellipsoid along the third axis of the ellipsoid may be smaller than 20 μm.

Furthermore, the examination apparatus and/or the method may also be used for examining channels in technical objects. In this case the beads may have a diameter up to 1 mm or even up to 1 cm.

For example, the permanently magnetic beads may be formed as spheres with a diameter of about 5 μm. In other words, the permanently magnetic beads may be sized to be able to circulate in channels having a diameter of about 5 μm. The beads can be simply ground permanently magnetic material such as Fe14Nd2B. Furthermore, the beads may have a shape such that they pass through channels having the size of human capillaries. It may further be desirable to maximize the volume of the beads. The beads may have a shape anisotropy. For example, the beads may have a prolate circular ellipsoid shape. The rotation frequency of such ellipsoid beads may be calculated as follows:

$$f = \gamma \,/\, (2\,\pi) \,*\, (e \,/\, (e^2 + 1))$$

where f is the rotation frequency of the bead, γ the shear rate of the fluid, and e the aspect ratio or eccentricity of the ellipse. Consequently, for growing eccentricities, the rotation frequency decreases. On the other hand, the rotation of the beads becomes more uneven and higher frequency components appear in the temporal magnetic field variations caused by the rotation of the ellipsoid beads when the eccentricity is growing. These higher frequency components of the magnetic field variations may allow a better discrimination between the magnetic field variations caused by the beads and the background noise, which background may be for example caused by the patient. This may be because the background noise is strongest at the lower frequencies, for example when the noise is mainly due to electric activity of the patient due to neural heart and brain activity. Generally speaking, the permanently magnetic beads may exhibit an isotropic shape in order to have an uneven rotation in the fluid.

The feature that the beads are permanently magnetic can be understood in the way that the beads have a non-zero residual magnetic dipole moment at zero external magnetic field. For example, the magnetic dipole moment of the beads may be greater than 50 pAm$^2$. For example, the magnetic dipole moment of the beads may be greater than 70 pAm$^2$. In other words, the permanently magnetic beads may be made out of a hard magnetic material.

The object may relate to any kind of object comprising a channel-like system where a fluid is flowing. For example, the object may be a human or animal body. However, the invention is not restricted to the examination of human or animal bodies and can relate to the examination of any kind of objects. The channel may relate to any kind of tube-like structure, in which a fluid is flowing. For example, the channel may be part of a cardiovascular system. The fluid may be a liquid, e.g. blood.

The examination zone may relate to any portion of the apparatus, in which the object may be placed for creating an image of the object. The magnetic sensors may be arranged such that magnetic fields within the examination zone may be detected. For example, the examination zone may comprise an examination table, on which the object may be placed. In other words, the examination zone may be a specific spatial area of the examination device, within which permanently magnetic beads can be tracked by the magnetic field sensors. The examination zone may be located such that the at least three magnetic field sensors at least partially surround the examination zone such that magnetic fields of beads located inside the examination zone can be detected.

The magnetic field sensor may relate to a sensor that is capable of detecting magnetic fields of permanently magnetic beads within the examination zone. In other words, the magnetic field sensor may be a magnetometer. The magnetic field may be caused by the permanently magnetic beads. Furthermore, the magnetic field may comprise background noise, which may be caused by a magnetic activity of the object itself. The magnetic field sensors may be adapted to magnetically identify the position of individual beads. Furthermore, the magnetic field sensor may be configured to measure magnetic field variations having frequencies between 1 Hz and 10 kHz. For example, the magnetic field sensor may be a superconducting quantum interference device (SQUID), an atomic magnetometer, and/or an induction coil. The magnetic field sensors of the examination apparatus may be arranged around or at least partly around the examination zone. For example, the magnetic field sensors may be placed on a circle segment around the examination zone.

The examination apparatus may for example comprise at least 10 magnetic field sensors. Preferably, the examination apparatus may comprise at least 30 magnetic field sensors. Even more preferred, the examination apparatus may comprise more than 100 magnetic field sensors. Furthermore, the examination apparatus may comprise not more than 300 magnetic field sensors. The number of magnetic field sensors may be optimized such that, at the same time, the number of signals to be processed by the evaluation unit is minimized and the resolution of the examination apparatus is maximized. Each magnetic field sensor may be configured for only measuring one field-component. In other words, the examination apparatus may comprise vector-sensors or 3d-sensors, wherein each vector-sensor/3d-sensor comprises 3 magnetic field sensors.

The evaluation unit may be any kind of processing unit that is configured for receiving the signal of the at least three magnetic field sensors and to process said signals. In particular, the evaluation unit may be configured for discriminating or separating sub-signals caused by individual or single beads from the overall signal generated by the at least three magnetic field sensors. The evaluation unit may be configured for discriminating the sub-signals using signal processing methods, wherein the signal processing methods are adapted in such a way that they are able to discriminate sub-signals that are due to temporal magnetic field variations caused by rotations of the individual beads. In other words, a sub-signal may describe a magnetic field variation that is caused by only one rotating permanently magnetic bead. Thus, the evaluation unit may be configured for separating such a sub-signal of a single rotating permanently magnetic bead from the overall signal generated by the magnetic field sensors and caused by the plurality of magnetic beads and eventually comprising background noise.

The rotation of the permanently magnetic beads may be due to the shear forces of the fluid flowing in the channel. For example, the shear rates in a channel tree may vary between 150 to 250 1/s in the aorta and may rise up to 1500 1/s in the arteriole. Furthermore, the shear rates may be around 1200 1/s in the capillaries and descend to 700 1/s in the venules. In the veins, the shear rates may vary between 150 1/s in the large veins and 5 1/s in the vena cava. A typical value for wall shear rates in the coronaries may be around 400 1/s. However, this value may exceed 10000 1/s in the case of stenosis. Because of these different shear rates, the frequencies of the magnetic field variations caused by the rotating magnetic beads may range between 0 Hz and 10 kHz.

The feature that the evaluation unit is configured for determining position information related to the individual beads shall mean that each position of each bead may be determined. For example, the position information may be determined during the procedure of fitting a model of the object to the measurements of the magnetic field sensors. For example, the position information may comprise coordinates of the individual beads. The position information of the individual beads may be determined on the basis of sensitivity encoding of the sub-signals related to individual beads. In other words, the position information may be determined with a procedure that is similar to triangulation of the permanently magnetic beads. Simply speaking, the different field strength measured by each one of the at least three magnetic field sensors may be used for localizing individual beads and/or for determining the position information of the individual beads. Furthermore, a degree of rotation asymmetry of the beads may be used for determining their position information. Some beads may, however, be indistinguishable (e.g. due to their symmetric position along the channel). Therefore, it shall be understood that the evaluation unit is configured for distinguishing individual beads and for determining their positions with a few exceptions that are not distinguishable.

Temporal magnetic field variations may relate to the variations of the magnetic field that are due to the rotation of the permanently magnetic beads. The overall magnetic field measured by the magnetic field sensors may comprise a superposition of a plurality of temporal magnetic field variations, wherein each variation is caused by a rotation of an individual bead. By separating the temporal magnetic field variations from each other, the sub-signals of each individual bead may be discriminated such that the position of each individual bead may be determined.

A model of the object may mathematically describe a magnetic field generated by permanently magnetic beads located in the channels of the object. The object may depend on a plurality of parameters which can be fitted to the magnetic field detected by the at least three magnetic field sensors such that the magnetic field described by the model of the object matches the magnetic field detected by the magnetic field sensors. In this way, position information of the individual beads may be determined. Moreover, the object and/or the channel may be reconstructed.

A measuring device configured for measuring a magnetic activity of the object may for example be a device for measuring a current flowing in the object or an electric potential of the object. The current flowing through the object, which current may be due to nervous activity, may cause a magnetic field that constitutes a background in the signal generated by the magnetic field sensors. In order to subtract this background from the signal of the magnetic field sensors, the magnetic activity of the object is determined, for example by measuring the current for electric potential. For example, the measuring device may be an electrocardiograph. The measuring device may be also another kind of measuring device that is capable of measuring a magnetic activity of the object which constitutes a background of the signal generated by the magnetic field sensors. Furthermore, also the magnetic sensors themselves may be the measuring device. In this case, the magnetic field sensors may be used for measuring the magnetic activity of the object when no beads are inserted in the object. Also a combination of the magnetic field sensors and an additional measuring device may be used for measuring the magnetic activity of the object.

The magnetic gradient field may relate to a magnetic gradient field that is generated by a magnetic device, for example an electromagnet or coil, of the examination apparatus. The magnetic gradient field may be an inhomogeneous magnetic field. In other words, the magnetic gradient field may be a magnetic gradient field with a gradient which is not constant. In particular, the magnetic gradient field may comprise a gradient within the field of view defined by the magnetic field. For example, the gradient may amount to about 1 mT/(m$\mu_0$). The magnetic gradient field may be used to enhance the sensitivity of the examination apparatus. In other words, the magnetic gradient field may cause an uneven rotation of the magnetic beads such that the discrimination or separation of the sub-signals of the individual magnetic beads may be enhanced.

The field of view may relate to a zone or region within the magnetic gradient field created by the magnetic device. Inside the field of view, the magnetic gradient field has such a strength that rotation of the magnetic beads is not inhibited for the given lowest sheer rate that is to be detected by the examination apparatus. Outside the field of view, the magnetic gradient field may have such a strength that the permanently magnetic beads do not rotate any more for the maximally expected sheer rate. There may be a rim in the field of view, where the detection probability of beads decreases. The field free point or field free line may relate to a point or a line within the magnetic gradient field where the field strength vanishes.

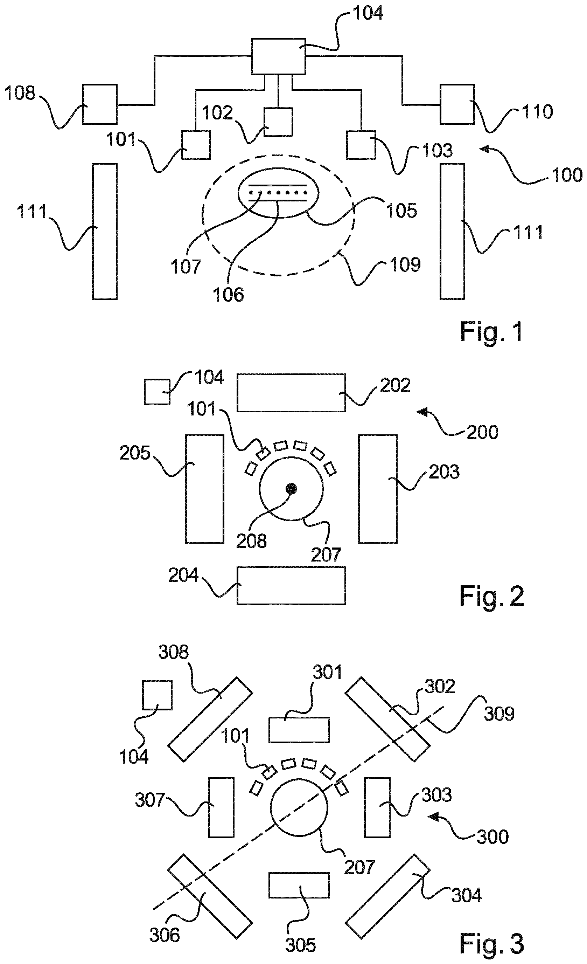

Fig. 1 shows an examination apparatus 100 for tracking a plurality of permanently magnetic beads 107 flowing in a channel 106 of an object 105 according to an exemplary embodiment of the invention. The object 105 is located in an examination zone 109 of the examination apparatus. The examination apparatus comprises at least three magnetic field sensors 101, 102 and 103 which are configured for detecting a magnetic field of the permanently magnetic beads in the examination zone and are configured for creating a corresponding signal. In other words, each of the at least three magnetic field sensors may be configured for detecting a magnetic field of the permanently magnetic beads and each of the at least three magnetic field sensors may be configured for creating a corresponding signal. The signal of the at least three magnetic field sensors may therefore relate to the entirety of the signals generated by each one of the at least three magnetic field sensors. Furthermore, the examination apparatus 100 comprises an evaluation unit 104 that is configured for determining temporal magnetic field variations by processing the signal created by the magnetic field sensor, wherein the temporal magnetic field variations are caused by rotations of the individual beads. Furthermore, the evaluation unit is configured for discriminating sub-signals caused by the individual beads in the processed signal using the determined temporal magnetic field variations. In other words, the evaluation unit may be configured for determining information about the temporal magnetic field variations and use this information for discriminating or separating the sub-signals. Moreover, the evaluation unit is configured for determining position information related to the individual beads using magnitudes of the sub-signals caused by the individual beads.

According to a further exemplary embodiment of the invention, the evaluation unit 104 may be configured to use the determined temporal magnetic field variations of the individual permanently magnetic beads 107 for determining the position information related to the individual beads. In other words, the evaluation unit may be configured for determining the position information related to the individual beads on the basis of the magnitudes of the sub-signals (i.e. the field strength of the magnetic field caused by the individual beads) and on additional information regarding the temporal magnetic field variations caused by the rotating permanently magnetic beads. This additional information regarding the temporal magnetic field variations may for example comprise frequency information and/or Fourier components of oscillations in the temporal magnetic field variations.

According to a further exemplary of the invention, the examination apparatus 100 may comprise a memory unit 108, wherein a model of the object is stored on the memory unit. Furthermore, the evaluation unit is configured for storing the signals of the at least three magnetic field sensors for different points in time on the memory unit. Moreover, the evaluation unit is configured for fitting the model to the signals of the at least three magnetic field sensors for different points in time stored on the memory unit. In other words, the model of the object may be fitted to the magnetic fields measured by the at least three magnetic field sensors over a certain period of time.

According to a further exemplary embodiment of the invention, the evaluation unit is configured to store current temporal magnetic field variations and/or determined position information of the beads as a history for next signals processing on the memory unit. Furthermore, the evaluation unit is configured to read the historical temporal magnetic field variations and/or position information of the beads stored on the memory unit. Moreover, the evaluation unit is configured to combine current temporal magnetic field variations and/or determined position information of the beads with historical temporal magnetic field variations and/or determined position information of the beads to retrieve the position or flow of each individual particle in the channel over time.

According to a further exemplary embodiment of the invention, the evaluation unit is configured to reconstruct the image of the channel based on the determined position information of beads which are identified to be located close to walls of the channel. Thus, the evaluation unit may be configured to determine whether a bead is located close to or at a wall of the channel.

According to a further exemplary embodiment of the invention, the examination apparatus 100 comprises a measuring device 110 that is configured for measuring a magnetic activity of the object 105 itself. Furthermore, the model of the object stored on the memory unit 108 is configured in such a way that it also depends on the magnetic activity of the object 106. Moreover, the evaluation unit is configured for fitting the model to the signals of the at least three magnetic field sensors 101, 102 and 103 and the measured magnetic activity of the object 105.

In other words, the evaluation unit may be configured for determining a background noise via the measuring device 110 and for subtracting said background noise from the signals generated by the magnetic field sensors 101, 102 and 103. In this way, the background noise caused by magnetic activity of the object itself may be subtracted such that positions of the individual beads may be determined more accurately.

According to a further exemplary embodiment of the invention, the evaluation unit 104 may be configured for subtracting the magnetic activity measured by the measuring device 110 from the signal generated by the magnetic field sensors 101, 102, and 103. In this way, the background noise or at least a part of it may be removed from the signal.

According to a further exemplary embodiment of the invention, each one of the at least three magnetic field sensors 101, 102, and 103 has a detection range of frequencies of temporal magnetic field variations in the range of 1 Hz to 10 kHz. Furthermore, the evaluation unit 104 is configured for discriminating the sub-signals caused by the individual beads in the processed signal using the determined temporal magnetic field variations having frequencies in the range of 1 Hz to 10 kHz. In this way, the examination apparatus may be adapted for discriminating permanently magnetic beads that are for example rotating in blood vessels.

According to a further exemplary embodiment of the invention, the at least three magnetic field sensor comprises a superconducting quantum interference device (SQUID), an atomic magnetometer and/or an induction coil. In this way, the examination apparatus comprises magnetic field sensors that are capable of detecting magnetic field variations caused by permanently magnetic beads rotating in blood vessels.

According to a further exemplary embodiment of the invention, the at least three magnetic field sensors comprise a plurality of magnetic field sensors of different types, wherein each type of magnetic field sensors is configured for detecting temporal magnetic field variations having a different range of frequencies. For example, the at least three magnetic field sensors may comprise SQUIDs and/or atomic magnetometers for the lower frequencies and one or more induction coils for the higher frequencies.

According to a further exemplary embodiment of the invention, the examination apparatus comprises a magnetic device 111 configured for creating a magnetic gradient field in the examination zone 109. More details regarding the magnetic device for creating a magnetic gradient field are shown and described with respect to Figs. 2 and 3.

Generally, it should be noted that the device and its components shown in Fig. 1 is presented in an illustrative way. In particular, the positioning of the components and the numbers thereof is simplified.

In Fig. 2, an examination apparatus 200 is shown. The examination apparatus comprises at least three magnetic field sensors 101, an evaluation unit 104 and four magnetic devices 202, 203, 204 and 205. Furthermore, the examination apparatus may comprise two additional magnetic devices which are positioned in front and in the back of the magnetic devices 202 to 204. In other words, the magnetic devices 202 to 205 and the two additional magnetic devices which are not shown may be positioned such that each magnetic device is located on a side of a cube or cuboid around the field of view 207. Each magnetic device may for example be an electromagnet or coil.

The arrangement of magnetic devices shown in Fig. 2 results in a magnetic gradient field having a field free point 208. However, there are also other arrangements of magnetic devices resulting in a magnetic gradient field having a field free point.

According to a further exemplary embodiment of the invention, the magnetic devices 202 to 205 may be configured for creating the magnetic gradient field such that the magnetic gradient field is varying in time. This may for example be achieved by varying the currents flowing through the magnetic devices (i.e. the electromagnets).

Fig. 3 shows another examination apparatus according to a further exemplary embodiment of the invention. The examination apparatus 300 comprises at least three magnetic field sensors 101, an evaluation unit 104 and a plurality of magnetic devices 301 to 308 (for example electromagnets or coils). In addition to the magnetic devices shown in Fig. 3, the examination apparatus may further comprise magnetic devices located in the front and the back of the apparatus 300 as described with respect to Fig. 2. The magnetic devices shown in Fig. 3 are arranged in such a way that they create a magnetic gradient field comprising a field free line 309. By controlling the currents applied to the single magnetic devices, the positioning of the field free line may be changed. Thus, the field free line may be rotated and/or translated, for example along Lissajous trajectories.

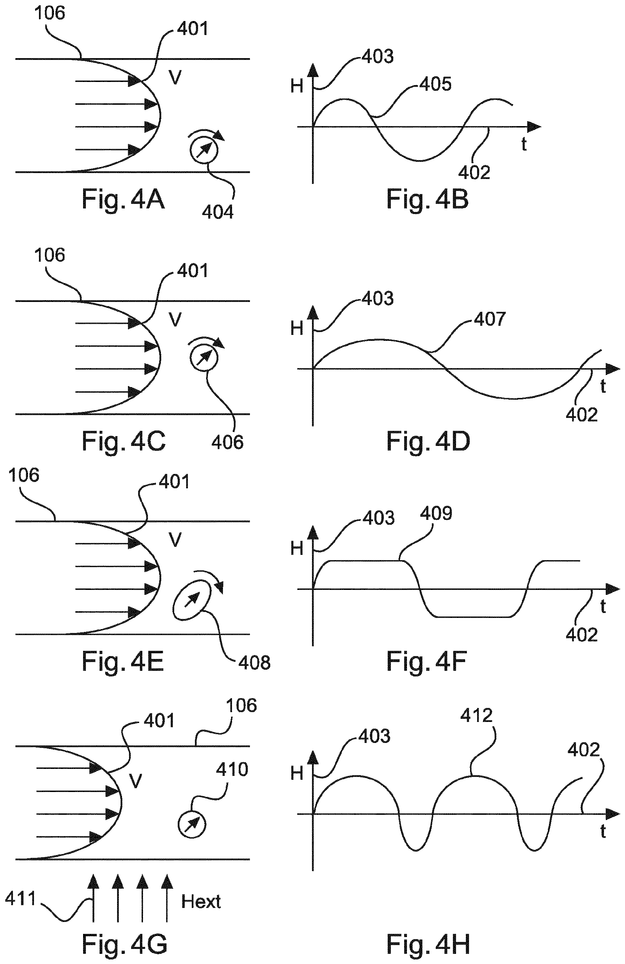

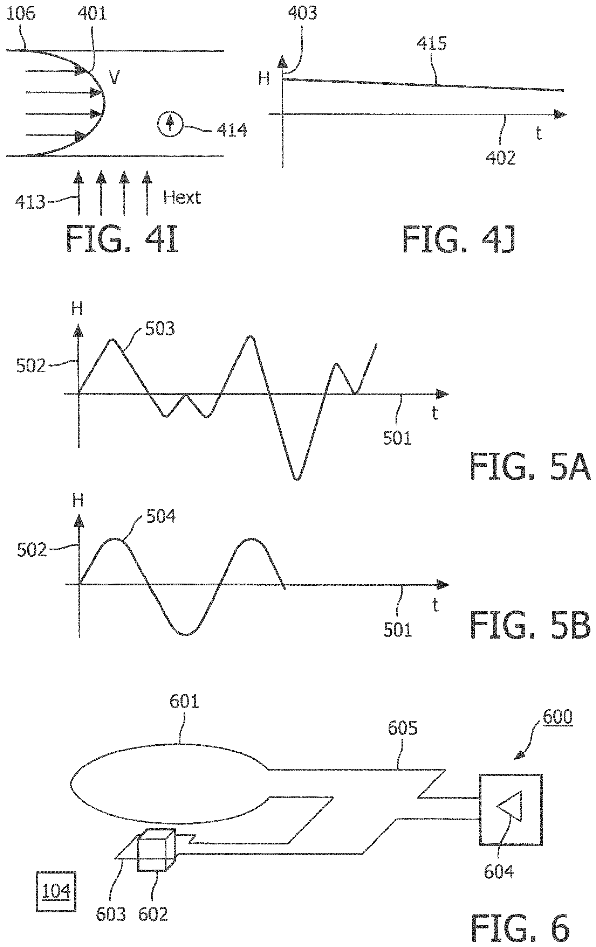

Figs. 4A to 4K each show a permanently magnetic bead within a channel and the corresponding magnetic field variations according to exemplary embodiments of the invention. In particular, Figs. 4A, 4C, 4E, 4G, and 4I show different configurations of permanently magnetic beads 404, 406, 408, 410, 414 and their positions in the channel 106. The channel 106 is flooded with a fluid having a velocity profile 401. Figs. 4B, 4D, 4F, 4H and 4K show the magnetic field variations 405, 407, 409, 412 and 415 corresponding to the situations shown in Figs. 4A, 4C, 4E, 4G and 4I. In Figs. 4B, 4D, 4F, 4H and 4K, graphs of the magnetic field variations are shown, wherein the horizontal axis 402 indicates time t and the vertical axis 403 indicates magnetic field strength H.

In Fig. 4A a first exemplary embodiment of the invention is shown where the permanently magnetic bead 404 is located relatively near the wall of the channel 106. At this position, the shear rates of the fluid are relatively high. This results in magnetic field variations 405 shown in Fig. 4B with a relatively high frequency. Ideally, the magnetic field variations 405 have a sinusoidal shape.

In Fig. 4C a further exemplary embodiment of the invention is shown where the permanently magnetic bead 406 is located more in the centre of the channel 106 compared to the situation shown in Fig. 4A. At this position, the shear rates of the fluid are not as large as at the position shown in Fig. 4A. Therefore, the temporal magnetic field variations 407 shown in Fig. 4D have a lower frequency as the ones shown in Fig. 4B.

In Fig. 4E another exemplary embodiment of the invention is shown where the permanently magnetic bead 408 has an ellipsoid shape and is located near the wall of the channel 106. Therefore, the frequency of the rotation is relatively high compared to the situation shown in Fig. 4C as can be seen in Fig. 4F. Furthermore, the magnetic field variations 409 have a shape that deviates considerably from the sinusoid shape since the ellipsoid is rotating unevenly.

According the exemplary embodiment of Fig. 4G it is shown that the permanently magnetic bead 410 is located near the wall of the channel 106. Furthermore, an external magnetic field $H_{ext}$ (e.g. a magnetic gradient field) is applied 411. In Fig. 4H it can be gathered, that the magnetic field variation 412 also deviate from the sinusoid shape shown in Figs. 4B and 4D. This is due to the fact that the external magnetic field $H_{ext}$ causes that one half of the full rotation of the permanently magnetic bead is finished much faster than the other half of the rotation. This is due to the fact that the magnetic field 411 defines a preferred alignment of the permanently magnetic bead.

In Fig. 4I a further exemplary embodiment is shown where the permanently magnetic bead is located near the wall of the channel 106 and that an external magnetic field $H_{ext}$ 413 (e.g. a magnetic gradient field) is applied which is stronger than the external magnetic field 411 shown in Fig. 4G. In this case, the permanently magnetic field 413 is so strong that the permanently magnetic bead 414 does not rotate any more. On the contrary, the permanently magnetic bead 414 is aligned in its preferred position defined by the external magnetic field 413. Consequently, it is shown that the magnetic field 415 of the permanently magnetic bead 414 does not oscillate any more. The slow decreasing of the magnetic field 415 is due to a slow translation of the permanently magnetic bead 414 due to the external magnetic field 413. However, this slow variation of the magnetic field 415 may not be detectable due to the noise background of the signal.

In Fig. 5A, a simplified signal 503 according to an exemplary embodiment of the invention is shown. Fig. 5A shows a diagram having a horizontal axis 501 relating to time t and a vertical axis 502 relating to magnetic field strength H. It can be gathered, that the signal 503 has a more complex shape which is due to the superimposition of different magnetic field variations caused by individual permanently magnetic beads.

In Fig. 5B, a sub-signal 504 comprised by the overall signal 503 is shown. By separating the sub-signal 504 from the overall signal 503, the position information of the permanently magnetic bead causing the sub-signal 504 can be determined by analyzing the field strength of the sub-signal 504 determined by the different magnetic field sensors. In other words, for every magnetic field sensor a sub-signal 504 for a single bead may be determined such that the plurality of sub-signals 504 for the single bead may be used for determining the position information of the single bead.

In Fig. 6, an examination apparatus 600 according to a further exemplary embodiment of the invention is shown. The examination apparatus 600 comprises a magnetic field sensor 602 which is for example a superconducting quantum interference device SQUID (the other magnetic field sensors of the examination apparatus are not shown). Furthermore, the examination apparatus 600 comprises an evaluation device 104 and a magnetic device 601, for example an electromagnet or coil. Furthermore, the examination apparatus 600 comprises an amplifier 604 for providing an electric current to the magnetic device 601, wherein the magnetic device 601 is connected to the amplifier 604 via the circuit 605. In order to compensate the magnetic field generated by the magnetic device 601 in the magnetic field sensor 602 (i.e. the SQUID or atomic magnetometer), an additional coil 603, which is connected to the same electric circuit as the magnetic device 601 is positioned around the magnetic field sensor 602. The additional coil 603 surrounding the magnetic field sensor 602 may be serially coupled to the circuit 605 of the magnetic device 601. There are also other possibilities to decouple the current from the circuit 605 into the coil 603, for example with a parallel circuit. If there are more than three magnetic field devices (e.g. electromagnet or coils) for which magnetic field compensation is needed, it may be preferable to use only three orthogonal compensation coils 603 on each magnetic field sensor and to decouple the electric current with parallel circuits from the circuit 605.

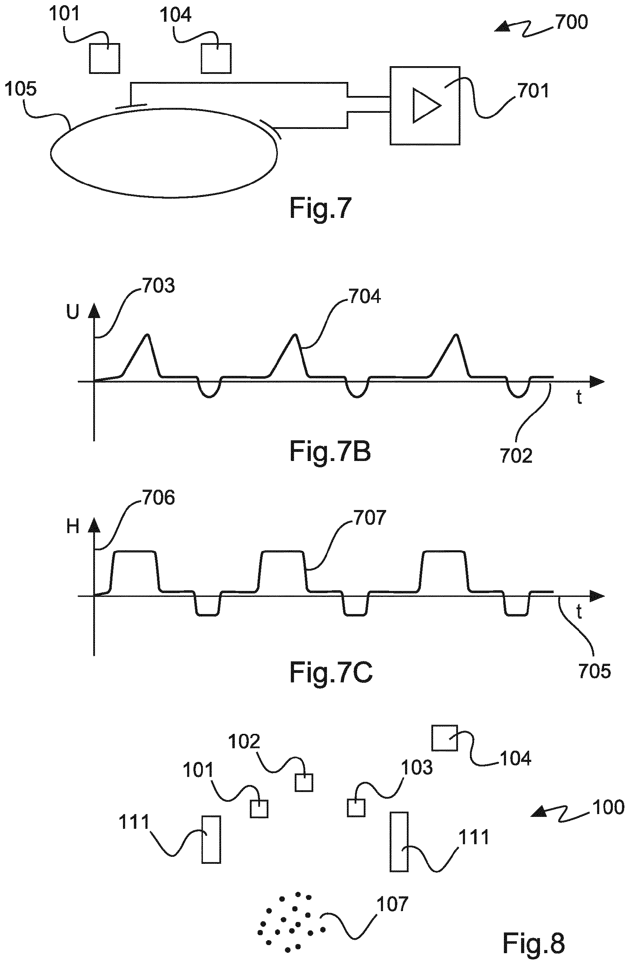

In Fig. 7A, an examination apparatus 700 according to a further exemplary embodiment of the invention is shown. The examination apparatus comprises a magnetic field sensor 101, an evaluation device 104 and a measurement device 701 for measuring a magnetic activity of the object 105. The magnetic activity may for example be determined by measuring a current and/or electric potential of the object 105. For example, the measurement device may be an electrocardiograph. Furthermore, the examination apparatus may comprise further components not shown in Fig. 7, e.g. magnetic devices and/or a memory unit.

In Fig. 7B, an electric potential measured by the measurement device 701 according to an exemplary embodiment of the invention is shown. The horizontal axis 702 relates to the time t and the vertical axis 703 to the electric potential U of the object. The curve 704 relates to the temporal variation of the electric potential in the object 105, for example the human body, which may be due to the nervous activity relating to the heartbeat. In Fig. 7C, the magnetic activity caused by the electric currents in the object is shown simultaneously recorded as Fig. 7B. The horizontal axis 705 relates to time and the vertical axis 706 to the magnetic field strength H. The curve 707 shows the variations of the magnetic activity of the object 105, for example the human body, that is due to the change of the electric potential in the object. As can be seen, the signals shown in Fig. 7B and 7C are strongly correlated to one another. In order to subtract the magnetic activity from the signal generated by the magnetic field sensors, a function may be determined, which reproduces the magnetic activity 707 from the electrical activity in the object. This function can be used to subtract the background from the signals generated by the magnetic field sensors 101.

Fig. 8 shows an examination apparatus 100 according to an exemplary embodiment of the invention. The examination apparatus 100 includes at least three magnetic field sensors 101, 102, and 103, an evaluation unit 104 and e.g. magnetic devices 111. Furthermore, the examination apparatus 100 comprises a plurality of permanently magnetic beads 107.

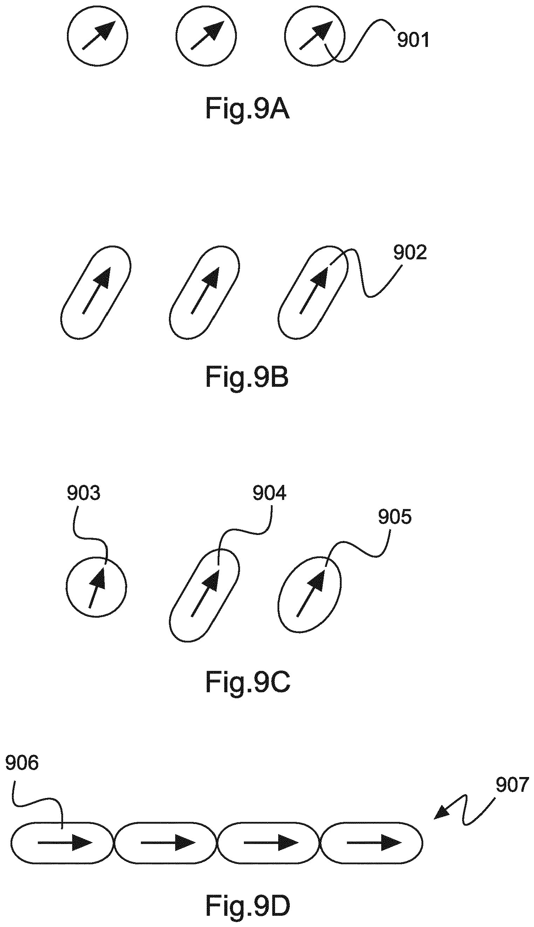

Figs. 9A to 9D each show a plurality of permanently magnetic beads according to exemplary embodiments of the invention.

According to an exemplary embodiment of the invention, Fig. 9A shows that the plurality of permanently magnetic beads comprises beads 901 that have an approximately spherical shape.

Fig. 9B shows according to a further exemplary embodiment of the invention that the permanently magnetic beads 902 have an ellipsoid shape.

In Fig. 9C, a further exemplary embodiment is shown, where the plurality of permanently magnetic beads comprises beads 903, 904, and 905 of different size and/or shape. For example, one bead 903 has a spherical shape, another bead 904 has an ellipsoid shape with large eccentricity, and a bead 905 has an ellipsoid shape with a smaller eccentricity.

Fig. 9D shows a further exemplary embodiment of the invention where the plurality of magnetic beads comprises a flexible chain 907 of single permanently magnetic beads 906 that are aligned behind each other. Such chains 907 may provide a stronger magnetic field variation and may still pass through small channels.

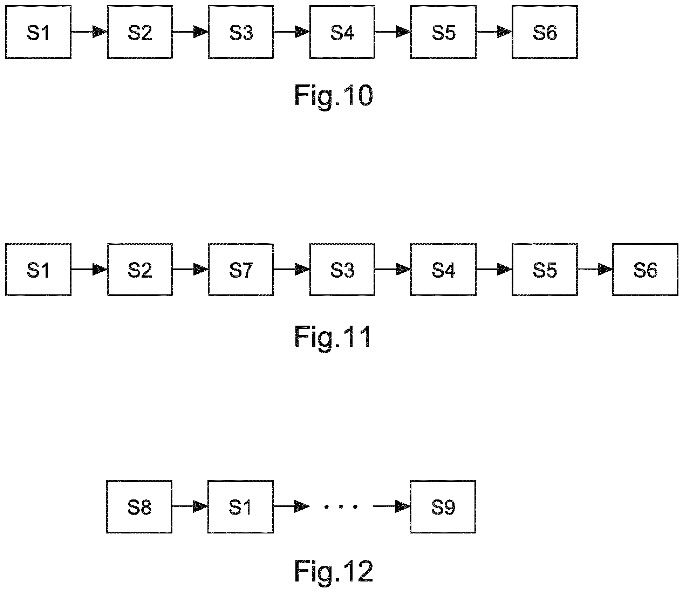

Fig. 10 shows a flow-chart of a method for tracking a plurality of permanently magnetic beads included in a fluid flowing through a channel of an object according to an exemplary embodiment of the invention. The method comprises step S2 of placing the object in an examination zone. Moreover, the method comprises step S3 of detecting the magnetic field of the permanently magnetic beads in the examination zone and generating a corresponding signal. The method also comprises step S4 of determining temporal magnetic field variations caused by individual beads by processing the signals, which temporal magnetic field variations are caused by the individual rotating magnetic beads. Furthermore, the method comprises step S5 of discriminating sub-signals caused by the individual beads in the processed signal using the determined temporal magnetic field variations of the individual beads. The method further comprises step S6 of determining position information related to the individual beads using magnitudes of the sub-signals caused by the individual beads. Optionally, the method comprises step S1 of inserting the plurality of permanently magnetic beads into the fluid flowing in the channel of the object, wherein the magnetic beads rotate when the fluid is flowing through the channel.

In Fig. 11, a flow-chart of a method for imaging a plurality of permanently magnetic beads according to another exemplary embodiment of the invention is shown. The method comprises the additional step S7 of generating a magnetic gradient field in the examination zone. According to an exemplary embodiment of the invention, the magnetic gradient field is generated in such a way that it is varying in time. According to a further exemplary embodiment of the invention, the magnetic gradient field may be created in such a way that it defines a field of view within the examination zone such that rotation of beads inside the field of view is possible and is inhibited outside the field of view. For example, by varying the magnetic gradient field in time, the field of view can be moved. In this way, a larger area of the object may be scanned. According to another exemplary embodiment of the invention, the magnetic field may comprise a field free point or a field free line. This additional step S7 of generating the magnetic gradient field may be carried out before and/or during the step S3 of detecting the magnetic field of the permanently magnetic bead in the examination zone and creating a corresponding signal.

In Fig. 12, another flow-chart for a method for imaging a plurality of permanently magnetic beads according to another exemplary embodiment of the invention is shown. The method further comprises step S8 of measuring a magnetic activity of the object itself, which is carried out before inserting the permanently magnetic beads within the channel or when a magnetic field is created that has such a strength such that it defines no field of view (e.g. when a homogenous magnetic field is created in the examination zone). Furthermore, the method comprises step S9 relating to fitting a model of the object, which model depends on the magnetic activity of the object, to the signals of the at least three magnetic field sensors and the measured magnetic activity of the object. According to an exemplary embodiment of the invention, step S9 relates to removing the measured magnetic activity from the signal generated by the magnetic field sensors. In this way, background noise is removed from the signal.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art and in practising the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored distributed on a suitable medium, such as an optical storage medium or a solid-state medium supplied together with or as part of other hardware, but may also be distributed in other forms, such via the internet or other wired or wireless telecommunication systems. Any reference signs in the claims should not be construed as limiting the scope. The scope of the present invention is defined by the claims.

## Claims

1. An imaging apparatus (100) for tracking a plurality of permanently magnetic beads (107) included in a fluid flowing in a channel (106) of an object (105) and for imaging the channel, the imaging apparatus comprising:

   at least three magnetic field sensors (101, 102, 103);
   an evaluation unit (104);
   an examination zone (109);
   wherein the examination zone is configured to receive the object;
   wherein the at least three magnetic field sensors are configured for detecting a magnetic field of the permanently magnetic beads in the examination zone and configured for creating a corresponding signal (503);
   wherein the evaluation unit is configured for

   - determining temporal magnetic field variations (405, 407, 409, 412) by processing the signal created by the at least three magnetic field sensors, which temporal magnetic field variations are caused by rotations of individual beads;
   - discriminating sub-signals (504) caused by the individual beads in the processed signal using the determined temporal magnetic field variations;
   - determining position information related to the individual beads using magnitudes of the sub-signals caused by the individual beads; and
   - reconstructing an image of the channel based on the determined position information of the individual beads.

2. Imaging apparatus according to claim 1, further comprising:
   a magnetic device (111) configured for creating a magnetic gradient field in the examination zone.

3. Imaging apparatus according to claim 2, wherein the magnetic device is configured for creating the magnetic gradient field in the examination zone such that the magnetic gradient field is varying in time.

4. Imaging apparatus according to claim 2 or 3, wherein the magnetic gradient field created by the magnetic device defines a field of view within the examination zone such that rotation of beads inside the field of view is possible and is inhibited outside the field of view.

5. Imaging apparatus according to any of claims 2 to 4; wherein the magnetic gradient field comprises a field free point (208) or a field free line (309).

6. Examination apparatus according to any one of the preceding claims, wherein the evaluation unit is configured to determine information regarding shear forces acting in the fluid flowing through the channel using information about the temporal magnetic field variations; and/or wherein the evaluation unit is configured to determine positions of the permanently-magnetic beads with respect to a wall of the object using information about the temporal magnetic field variations.

7. Imaging apparatus according to any of the preceding claims, further comprising a memory unit (108); wherein a model of the object is stored on the memory unit; wherein the evaluation unit is configured for storing the signals of the at least three magnetic field sensors for different points in time on the memory unit; wherein the evaluation unit is configured for fitting the model to the signals of the at least three magnetic field sensor for different points in time.

8. Imaging apparatus according to claim 7, further comprising:

   a measuring device (110) configured for measuring a magnetic activity of the object itself; wherein the model of the object stored on the memory unit depends on the magnetic activity of the object; and wherein the evaluation unit is configured for fitting the model stored on the memory unit to the signals of the at least three magnetic field sensor and the measured magnetic activity of the object.

9. Imaging apparatus according to any one of the preceding claims, wherein each one of the at least three magnetic field sensors has a detection range for frequencies of temporal magnetic field variations in the range of 1 Hz to 10 kHz; and wherein the evaluation unit is configured for discriminating the sub-signals caused by the individual beads in the processed signal using the determined temporal magnetic field variations having frequencies in the range of 1 Hz to 10 kHz.

10. Imaging apparatus according to any one of the preceding claims, wherein the at least three magnetic field sensors comprise a superconducting quantum interference device, an atomic magnetometer and/or an induction coil.

11. Imaging apparatus according to any one of the preceding claims, wherein the at least three magnetic field sensors comprise a plurality of magnetic field sensors of different types; and wherein each type of magnetic field sensors is configured for detecting temporal magnetic field variations with a different range of frequencies.

12. Imaging apparatus (100) according to the preceding claims, further comprising:

    a plurality of permanently magnetic beads; wherein, preferably, the plurality of permanent magnetic beads comprises beads of different size and/or shape; and wherein, preferably, the evaluation unit is configured for discriminating temporal magnetic field variations of the individual beads using that the individual beads with different size and/or shape rotate differently due to their different sizes and/or shapes.

13. A method for tracking a plurality of permanently magnetic beads included in a fluid flowing in a channel of an object and for imaging the channel, comprising the steps:

    placing the object in an examination zone (S2); detecting the magnetic field of the permanently magnetic beads in the examination zone and creating a corresponding signal (S3); determining temporal magnetic field variations of individual beads by processing the signal, which temporal magnetic field variations are caused by the individual rotating magnetic beads (S4); discriminating sub-signals caused by the individual beads in the processed signal using the determined temporal magnetic field variations of the individual beads (S5); determining position information related to the individual beads using magnitudes of the sub-signals caused by the individual beads (S6); and reconstructing an image of the channel based on the determined position information of the individual beads.

14. Method according to claim 13, further comprising the step: generating a magnetic gradient field in the examination zone (S7).

15. Computer program, which, when it is executed by a processor of an imaging apparatus, instructs the processor to carry out the method according to claims 13 or 14.

**Patentansprüche**

1. Abbildungseinrichtung (100) zum Nachverfolgen einer Vielzahl von permanentmagnetischen Kügelchen (107), die in einem Fluid enthalten sind, das in einem Kanal (106) eines Objekts (105) strömt, und zum Abbilden des Kanals, wobei die Abbildungseinrichtung umfasst:

   mindestens drei Magnetfeldsensoren (101, 102, 103);
   eine Auswerteeinheit (104);
   einen Untersuchungsbereich (109);
   wobei der Untersuchungsbereich konfiguriert ist, um das Objekt aufzunehmen;
   wobei die mindestens drei Magnetfeldsensoren zum Erfassen eines Magnetfeldes der permanentmagnetischen Kügelchen im Untersuchungsbereich und zum Erzeugen eines entsprechenden Signals (503) konfiguriert sind;
   wobei die Auswerteeinheit konfiguriert ist zum

      - Bestimmen von zeitlichen Magnetfeldschwankungen (405, 407, 409, 412) durch Verarbeiten des von den mindestens drei Magnetfeldsensoren erzeugten Signals, wobei diese zeitlichen Magnetfeldschwankungen durch Drehungen einzelner Kügelchen verursacht werden;
      - Unterscheiden von Teilsignalen (504), die durch die einzelnen Kügelchen in dem verarbeiteten Signal verursacht werden, mithilfe der bestimmten zeitlichen Magnetfeldänderungen;
      - Bestimmen von Positionsinformationen in Bezug auf die einzelnen Kügelchen mithilfe von Größen der durch die einzelnen Kügelchen verursachten Teilsignale; und
      - Rekonstruieren einer Abbildung des Kanals basierend auf den bestimmten Positionsinformationen der einzelnen Kügelchen.

2. Abbildungseinrichtung nach Anspruch 1, weiter umfassend:
   eine magnetische Vorrichtung (111), die zum Erzeugen eines magnetischen Gradientenfeldes im Untersuchungsbereich konfiguriert ist.

3. Abbildungseinrichtung nach Anspruch 2,

wobei die magnetische Vorrichtung konfiguriert ist, um das magnetische Gradientenfeld im Untersuchungsbereich so zu erzeugen, dass das magnetische Gradientenfeld zeitlich variiert.

4. Abbildungseinrichtung nach Anspruch 2 oder 3, wobei das von der magnetischen Vorrichtung erzeugte magnetische Gradientenfeld ein Sichtfeld innerhalb des Untersuchungsbereichs definiert, so dass eine Drehung der Kügelchen innerhalb des Sichtfeldes möglich ist und außerhalb des Sichtfeldes verhindert wird.

5. Abbildungseinrichtung nach einem der Ansprüche 2 bis 4;
   wobei das magnetische Gradientenfeld einen feldfreien Punkt (208) oder eine feldfreie Linie (309) umfasst.

6. Untersuchungseinrichtung nach einem der vorstehenden Ansprüche,
   wobei die Auswerteeinheit konfiguriert ist, um Informationen über Scherkräfte zu bestimmen, die in dem durch den Kanal strömenden Fluid wirken, mithilfe von Informationen über die zeitlichen Magnetfeldänderungen; und/oder
   wobei die Auswerteeinheit konfiguriert ist, um Positionen der permanentmagnetischen Kügelchen in Bezug auf eine Wand des Objekts mithilfe von Informationen über die zeitlichen Magnetfeldänderungen zu bestimmen.

7. Abbildungseinrichtung nach einem der vorstehenden Ansprüche, weiter umfassend
   eine Speichereinheit (108);
   wobei ein Modell des Objekts auf der Speichereinheit gespeichert ist;
   wobei die Auswerteeinheit konfiguriert ist zum Speichern der Signale der mindestens drei Magnetfeldsensoren für verschiedene Zeitpunkte auf der Speichereinheit;
   wobei die Auswerteeinheit konfiguriert ist, um das Modell an die Signale des mindestens drei Magnetfeldsensors für verschiedene Zeitpunkte anzupassen.

8. Abbildungseinrichtung nach Anspruch 7, weiter umfassend:

   eine Messvorrichtung (110), die zum Messen einer magnetischen Aktivität des Objekts selbst konfiguriert ist;
   wobei das Modell des auf der Speichereinheit gespeicherten Objekts von der magnetischen Aktivität des Objekts abhängt; und
   wobei die Auswerteeinheit konfiguriert ist, um das auf der Speichereinheit gespeicherte Modell an die Signale des mindestens drei Magnetfeldsensors und die gemessene magnetische Aktivität des Objekts anzupassen.

9. Abbildungseinrichtung nach einem der vorstehenden Ansprüche, wobei jeder der mindestens drei Magnetfeldsensoren einen Erfassungsbereich für Frequenzen von zeitlichen Magnetfeldschwankungen im Bereich von 1 Hz bis 10 kHz aufweist; und wobei die Auswerteeinheit konfiguriert ist, um die durch die einzelnen Kügelchen in dem verarbeiteten Signal verursachten Teilsignale mithilfe der bestimmten zeitlichen Magnetfeldschwankungen mit Frequenzen im Bereich von 1 Hz bis 10 kHz zu unterscheiden.

10. Abbildungseinrichtung nach einem der vorstehenden Ansprüche, wobei die mindestens drei Magnetfeldsensoren eine supraleitende Quanten-Interferenz-Vorrichtung, ein atomares Magnetometer und/oder eine Induktionsspule umfassen.

11. Abbildungseinrichtung nach einem der vorstehenden Ansprüche, wobei die mindestens drei Magnetfeldsensoren eine Vielzahl von Magnetfeldsensoren verschiedener Typen umfassen; und wobei jede Art von Magnetfeldsensoren konfiguriert ist, um zeitliche Magnetfeldänderungen mit einem unterschiedlichen Frequenzbereich zu erfassen.

12. Abbildungseinrichtung (100) gemäß den vorstehenden Ansprüche, weiter umfassend:

eine Vielzahl von permanentmagnetischen Kügelchen; wobei die Vielzahl von permanentmagnetischen Kügelchen vorzugsweise Kügelchen unterschiedlicher Größe und/oder Form umfasst; und wobei die Auswerteeinheit vorzugsweise konfiguriert ist, um zeitliche Magnetfeldschwankungen der einzelnen Kügelchen zu unterscheiden, wobei sich die einzelnen Kügelchen mit unterschiedlicher Größe und/oder Form aufgrund ihrer unterschiedlichen Größen und/oder Formen unterschiedlich drehen.

13. Verfahren zum Nachverfolgen einer Vielzahl von permanentmagnetischen Kügelchen, die in einem Fluid enthalten sind, das in einem Kanal eines Objekts fließt, und zum Abbilden des Kanals, umfassend die Schritte:

Platzieren des Objekts in einem Untersuchungsbereich (S2); Erfassen des Magnetfeldes der permanentmagnetischen Kügelchen im Untersuchungsbereich und Erzeugen eines entsprechenden Signals (S3); Bestimmen von zeitlichen Magnetfeldschwankungen einzelner Kügelchen durch Verarbeiten des Signals, welche zeitlichen Magnetfeldschwankungen durch die einzelnen rotierenden Magnetkügelchen (S4) verursacht werden; Unterscheiden von Teilsignalen, die durch die einzelnen Perlen im verarbeiteten Signal verursacht werden, unter Verwendung der bestimmten zeitlichen Magnetfeldschwankungen der einzelnen Kügelchen (S5); Bestimmen von Positionsinformationen in Bezug auf die einzelnen Kügelchen unter Verwendung von Größen der durch die einzelnen Kügelchen verursachten Teilsignale (S6); und Rekonstruieren eines Abbildes des Kanals basierend auf den bestimmten Positionsinformationen der einzelnen Kügelchen.

14. Verfahren nach Anspruch 13, weiter umfassend den Schritt:

Erzeugen eines magnetischen Gradientenfeldes im Untersuchungsbereich (S7).

15. Computerprogramm, das, wenn es von einem Prozessor einer Abbildungseinrichtung ausgeführt wird, den Prozessor anweist, das Verfahren nach den Ansprüchen 13 oder 14 durchzuführen.

**Revendications**

1. Appareil d'imagerie (100) pour effectuer le suivi d'une pluralité de billes en permanence magnétiques (107) incluses dans un fluide s'écoulant dans un canal (106) d'un objet (105) et imager le canal, l'appareil d'imagerie comprenant :

au moins trois capteurs de champ magnétique (101, 102, 103) ; une unité d'évaluation (104) ; une zone d'examen (109) ; dans lequel la zone d'examen est configurée pour recevoir l'objet ; dans lequel les au moins trois capteurs de champ magnétique sont configurés pour détecter un champ magnétique de billes en permanence magnétiques dans la zone d'examen et configurés pour créer un signal correspondant (503) ; dans lequel l'unité d'évaluation est configurée pour :

- déterminer des variations temporelles de champ magnétique (405, 407, 409, 412) en traitant le signal créé par les au moins trois capteurs de cham magnétique, lesquelles variations temporelles de champ magnétique sont provoquées par des rotations de billes individuelles ;
- discriminer des sous-signaux (504) provoqués par les billes individuelles dans le signal traité en utilisant les variations temporelles déterminées du champ magnétique ;
- déterminer des informations de positions relatives aux billes individuelles en utilisant les grandeurs des sous-signaux provoqués par les billes individuelles ; et
- reconstruire une image du canal sur la base des informations de positions déterminées des billes individuelles.

2. Appareil d'imagerie selon la revendication 1, comprenant en outre ;
un dispositif magnétique (111) configuré pour créer un champ de gradient magnétique dans la zone d'examen.

3. Appareil d'imagerie selon la revendication 2, dans lequel le dispositif magnétique est configuré pour créer le champ de gradient magnétique dans la zone d'examen de sorte que le champ de gradient magnétique varie dans le temps.

4. Appareil d'imagerie selon la revendication 2 ou 3, dans lequel le champ de gradient magnétique créé par le dispositif magnétique définit un champ de vision dans la zone d'examen de sorte que la rotation de billes dans le champ de vision soit possible et soit inhibé en dehors du champ de vision.

5. Appareil d'imagerie selon l'une quelconque des revendications 2 à 4, dans lequel le champ de gradient magnétique comprend un point sans champ (208) ou une ligne sans champ (309).

6. Appareil d'imagerie selon l'une quelconque des revendications précédentes, dans lequel l'unité d'évaluation est configurée pour déterminer des informations concernant les forces de cisaillement agissant dans le fluide s'écoulant à travers le canal en utilisant des informations sur les variations temporelles de champ magnétique ; et/ou dans lequel l'unité d'évaluation est configurée pour déterminer des positions des billes en permanence magnétiques par rapport à une paroi de l'objet en utilisant des informations sur les variations temporelles de champ magnétique.

7. Appareil d'imagerie selon l'une quelconque des revendications précédentes comprenant en outre :

une unité de mémoire (108) ;
dans lequel un modèle de l'objet est stocké sur l'unité de mémoire ;
dans lequel l'unité d'évaluation est configurée pour stocker les signaux des au moins trois capteurs de champ magnétique pour différents points dans le temps sur l'unité de mémoire ;
dans lequel l'unité d'évaluation est configurée pour ajuster le modèle aux signaux des au moins trois capteurs de champ magnétique pour différents points dans le temps.

8. Appareil d'imagerie selon la revendication 7 comprenant en outre :

un dispositif de mesure (110) configuré pour mesurer une activité magnétique de l'objet lui-même ;
dans lequel le modèle de l'objet stocké sur l'unité de mémoire dépend de l'activité magnétique de l'objet ; et
dans lequel l'unité d'évaluation est configurée pour ajuster le modèle stocké sur l'unité de mémoire aux signaux des au moins trois capteurs de champ magnétique et à l'activité magnétique mesurée de l'objet.

9. Appareil d'imagerie selon l'une quelconque des revendications précédentes, dans lequel chacun des au moins trois capteurs de champ magnétique a une plage de détection pour des fréquences de variations temporelles de champ magnétique dans la plage de 1 Hz à 10 kHz ; et dans lequel l'unité d'évaluation est configurée pour discriminer les sous-signaux provoqués par les billes individuelles dans le signal traité en utilisant les variations temporelles déterminées de champ magnétique ayant des fréquences dans la plage de 1 Hz à 10 kHz.

10. Appareil d'imagerie selon l'une quelconque des revendications précédentes, dans lequel les au moins trois capteurs de champ magnétique comprennent un dispositif d'interférence de quantum supraconducteur, un magnétomètre atomique et/ ou une bobine d'induction.

11. Appareil d'imagerie selon l'une quelconque des revendications précédentes, dans lequel les au moins trois capteurs de champ magnétique comprennent une pluralité de capteurs de champ magnétique de différents types ; et dans lequel chaque type de capteur de champ magnétique est configuré pour détecter des variations temporelles de champ magnétique avec une plage différente de fréquences.

12. Appareil d'imagerie (100) selon les revendications précédentes, comprenant en outre :

une pluralité de billes en permanence magnétiques ;

dans lequel, de préférence, la pluralité de billes en permanence magnétiques comprend des billes de différentes tailles et/ou formes ; et

dans lequel, de préférence, l'unité d'évaluation est configurée pour discriminer des variations temporelles de champ magnétique des billes individuelles du fait que les billes individuelles de différentes tailles et/ou formes tournent différemment en raison de leurs différentes tailles et/ou formes.

13. Procédé pour effectuer le suivi d'une pluralité de billes en permanence magnétiques incluses dans un fluide s'écoulant dans un canal d'un objet et imager le canal, comprenant les étapes consistant à :

placer l'objet dans une zone d'examen (S2) ;

détecter le champ magnétique de billes en permanence magnétiques dans la zone d'examen et créer un signal correspondant (S3) ;

déterminer des variations temporelles de champ magnétique de billes individuelles en traitant le signal, lesquelles variations temporelles de champ magnétique sont provoquées par les billes magnétiques individuelles (S4) qui tournent ;

discriminer les sous-signaux provoqués par les billes individuelles dans le signal traité en utilisant les variations temporelles déterminées de champ magnétique des billes individuelles (S5) ;

déterminer des informations de positions relatives aux billes individuelles en utilisant des grandeurs des sous-signaux provoqués par les billes individuelles (S6) ; et

reconstruire une image du canal sur la base des informations de positions déterminées des billes individuelles.

14. Procédé selon la revendication 13, comprenant en outre l'étape consistant à :
générer un champ de gradient magnétique dans la zone d'examen (S7).

15. Programme d'ordinateur qui, lorsqu'il est exécuté par un processeur d'un appareil d'imagerie, instruit le processeur d'effectuer le procédé selon la revendication 13 ou 14.

Fig. 1

Fig. 2

Fig. 3

Fig. 4A

Fig. 4B

Fig. 4C

Fig. 4D

Fig. 4E

Fig. 4F

Fig. 4G

Fig. 4H

FIG. 4I

FIG. 4J

FIG. 5A

FIG. 5B

FIG. 6

101      104            700

105                          701

Fig.7

U  703        704

t
Fig.7B        702

H  706        707

t
Fig.7C        705

102      104
101  103
111      111  100

107
Fig.8

Fig.9A

901

Fig.9B

902

903 904 905

Fig.9C

906 907

Fig.9D

EP 3 370 612 B1

S1 → S2 → S3 → S4 → S5 → S6

Fig.10

S1 → S2 → S7 → S3 → S4 → S5 → S6

Fig.11

S8 → S1 → · · · → S9

Fig.12

22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080204009 A1
- US 20070172890 A1

- US 20130157256 A1

**Non-patent literature cited in the description**

- Model-Based Reconstruction for Magnetic Particle Imaging. **KNOPP T. et al.** IEEE Transaction On Medical Imaging. IEEE Service Center, 01 January 2010, vol. 29, 12-18